# FASCICULE DE BREVET EUROPEEN

(11) **EP 2 280 919 B1**
(45) Date de publication et mention de la délivrance du brevet: **31.07.2013**
(21) Numéro de dépôt: 09769505.0
(22) Date de dépôt: 29.05.2009
(51) Int. Cl.: C07C 45/52, C07C 47/22

(54) **PROCEDE DE FABRICATION D'ACROLEINE PAR DESHYDRATATION DU GLYCEROL**
VERFAHREN ZUR HERSTELLUNG VON ACROLEIN DURCH DEHYDRIERUNG VON GLYCEROL
METHOD FOR PRODUCING ACROLEIN BY MEANS OF DEHYDRATION OF GLYCEROL

(30) Priorité: 03.06.2008 FR 0853668
(43) Date de publication de la demande: 09.02.2011
(73) Titulaire: Arkema France, 92700 Colombes (FR)
(72) Inventeur: DUBOIS, Jean-Luc, F-69390 Millery (FR)
(74) Mandataire: Bonnel, Claudine
(86) Numéro de dépôt international: PCT/FR2009/051019
(87) Numéro de publication internationale: WO 2009/156664

(56) Documents cités:
- WO-A-2006/087083

## Description

La présente invention concerne un procédé perfectionné de fabrication d'acroléine par déshydratation du glycérol en présence d'additifs gazeux acides.

L'acroléine est le plus simple des aldéhydes insaturés. On l'appelle aussi 2-propenal, acrylaldéhyde ou aldéhyde acrylique. De par sa structure, l'acroléine a un pouvoir réactif élevé grâce à la présence de ses deux fonctions réactives qui sont susceptibles de réagir individuellement ou ensemble. C'est pourquoi, l'acroléine trouve de nombreuses applications, notamment comme intermédiaire de synthèse. C'est en particulier un intermédiaire clé pour la synthèse de la méthionine, acide aminé de synthèse utilisée comme complément de l'alimentation animale qui s'est imposé comme substitut des farines de poisson. L'acroléine est un intermédiaire de synthèse non isolé de l'acide acrylique dans la production industrielle de l'acide acrylique par oxydation catalytique du propylène en phase gazeuse. On connaît l'importance de la chimie de l'acide acrylique et de ses dérivés. L'acroléine conduit aussi par réaction avec le méthyl vinyl éther puis hydrolyse, au glutaraldéhyde qui possède de nombreuses utilisations dans le tannage du cuir, comme biocide dans les forages pétroliers et lors du traitement des huiles de coupe, et comme désinfectant et stérilisant chimique pour le matériel hospitalier.

Le procédé de production d'acroléine le plus communément utilisé est basé sur la réaction d'oxydation catalytique en phase gazeuse du propylène par l'oxygène de l'air. L'acroléine ainsi obtenue peut alors être directement intégrée dans un procédé de fabrication d'acide acrylique. Lorsque l'acroléine sert de matière première pour la synthèse de la méthionine et/ou d'acide acrylique et/ou d'acrylonitrile ou pour des réactions de chimie fine, une section de purification permet d'éliminer les sous-produits de la réaction, principalement les oxydes de carbone, l'acide acrylique, l'acide acétique et l'acétaldéhyde.

La production d'acroléine est donc fortement dépendante de la matière première propylène obtenu par vapocraquage ou craquage catalytique de coupes pétrolières. Cette matière première, d'origine fossile, contribue de plus, à l'augmentation de l'effet de serre. Il apparaît donc nécessaire de disposer d'un procédé de synthèse d'acroléine non dépendant de la ressource en propylène et utilisant une autre matière première, de préférence renouvelable. Ce procédé serait particulièrement avantageux pour la synthèse de la méthionine et autres produits, qui pourrait alors être dite "obtenue à partir de biomasse". En effet, la méthionine lors de son utilisation dans l'alimentation animale, est métabolisée rapidement et le gaz carbonique qui se retrouve dans l'atmosphère contribue à l'augmentation de l'effet de serre. Si l'acroléine est obtenue à partir d'une matière première renouvelable, obtenue par exemple à partir d'huile végétale, les émissions de CO₂ n'entrent plus dans le bilan du procédé, car elles compensent le gaz carbonique utilisé par la biomasse pour sa croissance ; il n'y a donc pas d'augmentation de l'effet de serre. Un tel procédé répond alors aux critères associés au nouveau concept de "chimie verte" dans un cadre plus global de développement durable.

11 est également connu de synthétiser des aldéhydes tels que l'acroléine par déshydratation d'un polyalcool tel que le glycérol. Le glycérol (appelé aussi glycérine quand il est sous forme de solution aqueuse) est notamment issu de la méthanolyse des huiles végétales et animales en même temps que les esters méthyliques qui sont, eux, employés notamment comme carburants ou combustibles dans le gazole et le fioul domestique. C'est un produit naturel, disponible en grande quantité, il peut être stocké et transporté sans difficulté. Il présente l'intérêt d'être une matière première renouvelable répondant aux critères associés au nouveau concept de "chimie verte".

De nombreuses études récentes sont consacrées à la valorisation du glycérol et notamment à la préparation de l'acroléine. Le procédé objet de ces études met en oeuvre une réaction de déshydratation du glycérol selon les réactions consécutives :

CH₂OH-CHOH-CH₂OH → CH₂OH-CH₂-CHO+ H₂O ⇔ CH₂=CH-CHO + 2H₂O

qui permettent d'obtenir l'acroléine.

Cette réaction est une réaction équilibrée ; en règle générale la réaction d'hydratation est favorisée aux basses températures, et la déshydratation est favorisée aux températures élevées. Pour obtenir l'acroléine, il faut donc mettre en oeuvre une température suffisante, et/ou un vide partiel pour déplacer la réaction. La réaction peut être effectuée en phase liquide ou en phase gaz. Ce type de réaction est connu pour être catalysée par des acides.

Selon le brevet FR 69.5931, on obtient l'acroléine en faisant passer des vapeurs de glycérine à une température suffisamment haute sur des sels d'acides ayant au moins trois fonctions acides comme par exemple des sels d'acide phosphorique. Les rendements indiqués sont supérieurs à 75% après distillation fractionnée.

Dans le brevet US 2,558,520, la réaction de déshydratation est faite en phase gaz/liquide en présence de terres de diatomées imprégnées de sels d'acide phosphorique, en suspension dans un solvant aromatique. Un taux de conversion du glycérol en acroléine de 72,3% est obtenu dans ces conditions.

Le procédé décrit dans la demande WO 99/05085 est basé sur une catalyse homogène complexe, sous atmosphère CO/H₂ sous pression 20/40 bars et en présence d'un solvant tel qu'une solution aqueuse de sulpholane.

La demande de brevet chinois CN 1394839 porte sur un procédé de préparation du 3-hydroxypropanaldéhyde à partir de glycérol. L'acroléine, produit intermédiaire de la réaction, est obtenue en faisant passer du glycérol pur vaporisé sur un catalyseur de type sulfate de potassium ou sulfate de magnésium, puis l'acroléine obtenue est réhydratée en hydroxypropanaldéhyde. Les rendements de la réaction ne sont pas donnés.

Le brevet US 5,387,720 décrit un procédé de production d'acroléine par déshydratation du glycérol, en phase liquide ou en phase gaz sur des catalyseurs solides acides définis par leur acidité de Hammett. Les catalyseurs doivent avoir une acidité de Hammett inférieure à +2 et de préférence inférieure à -3. Ces catalyseurs correspondent par exemple à des matériaux siliceux naturels ou de synthèse, comme la mordénite, la montmorillonite, les zéolithes acides ; des supports, tels que des oxydes ou des matériaux siliceux, par exemple l'alumine (Al₂O₃), l'oxyde de titane (TiO₂), recouverts par des acides inorganiques, mono, di ou triacides ; des oxydes ou oxydes mixtes tels que l'alumine gamma, l'oxyde mixte ZnO-Al₂O₃, ou encore des hétéropolyacides. Selon ce brevet, une solution aqueuse comprenant de 10 à 40% de glycérol est utilisée et on opère à des températures comprises entre 180° et 340°C en phase liquide et entre 250° et 340°C en phase gaz. Selon les auteurs de ce brevet, la réaction en phase gaz est préférable car elle permet d'avoir un taux de conversion du glycérol proche de 100 %, qui conduit à une solution aqueuse d'acroléine contenant des produits secondaires. Une proportion d'environ 10% du glycérol est convertie en hydroxypropanone qui se retrouve comme sous-produit majoritaire dans la solution d'acroléine. L'acroléine est récupérée et purifiée par distillation ou condensation fractionnée. Pour une réaction en phase liquide, une conversion limitée à 15 - 25 % est recherchée, pour éviter une perte trop importante de sélectivité. Le brevet US 5,426,249 décrit le même procédé en phase gaz de déshydratation du glycérol en acroléine, mais suivi d'une hydratation de l'acroléine et d'une hydrogénation pour conduire aux 1,2- et 1,3-propane diol.

La réaction de déshydratation du glycérol en acroléine s'accompagne ainsi généralement de réactions secondaires entraînant la formation de sous-produits tels que l'hydroxypropanone, le propanaldéhyde, l'acétaldéhyde, l'acétone, des produits d'addition de l'acroléine sur le glycérol (appelés acétals), des produits de polycondensation du glycérol, des éthers de glycérol cycliques..., mais aussi de phénol et des composés polyaromatiques qui sont à l'origine de la formation de coke sur le catalyseur. Il en résulte, d'une part une diminution du rendement et de la sélectivité en acroléine, d'autre part une désactivation du catalyseur. La présence des sous-produits dans l'acroléine, tels que l'hydroxypropanone ou le propanaldéhyde, certains étant de plus difficiles à isoler, nécessite des étapes de séparation et purification qui conduisent à des coûts élevés de récupération de l'acroléine purifiée. Par ailleurs, il est nécessaire de régénérer le catalyseur très souvent de façon à retrouver une activité catalytique satisfaisante.

La société déposante a cherché à résoudre ces problèmes en proposant dans le brevet français publié sous le n° 2.882.052 de conduire la réaction de déshydratation du glycérol en présence d'oxygène moléculaire. Il a été observé à cette occasion que de façon surprenante l'apport d'oxygène réduit la formation de composés aromatiques comme le phénol, et de sous-produits provenant d'une hydrogénation de produits déshydratés comme le propanaldéhyde et l'acétone, mais aussi de l'hydroxypropanone. La formation de coke sur le catalyseur se trouve réduite. Il en résulte une inhibition de la désactivation du catalyseur et une régénération en continu du catalyseur. Certains sous-produits se trouvent présents en quantités nettement plus faibles ce qui facilite les étapes ultérieures de purification.

Pour intéressants qu'ils soient, ces résultats ne sont pas suffisants sur le plan économique pour passer à une échelle industrielle. D'autre part la mise en oeuvre du procédé en présence d'oxygène implique des précautions opératoires afin d'éviter qu'elle ne s'emballe par aller jusqu' à la combustion avec ses risques d'explosion. Cela entraîne par exemple l'utilisation d'un gaz inerte pour rester en dehors de la zone d'inflammabilité. L'azote de l'air peut constituer une partie de ce gaz inerte mais sera souvent en quantité insuffisante ce qui amènera à utiliser des gaz inertes additionnels tels que les gaz de recycle contenant outre l'azote qui n'a pas pu réagir, les gaz de combustion et des gaz rares comme l'Argon, mais aussi des gaz volontairement ajoutés tels que les gaz cités ci-dessus mais aussi du méthane et des alcanes légers. L'utilisation de gaz inertes et qui, par définition ne contribuent pas à la réaction, implique l'utilisation d'un réacteur de grande taille, par rapport à ce qui est nécessaire aux seuls réactifs. Ceci entraîne un surcoût. C'est la raison pour laquelle la société déposante a poursuivi ses travaux pour améliorer la sélectivité en acroléine de la réaction en se focalisant sur les conditions d'efficacité et/ou de sélectivité des catalyseurs déjà connus pour être utilisés pour la synthèse de l'acroléine à partir du glycérol.

La société déposante a découvert avec surprise que les catalyseurs de type acide connus pour la catalyse de la réaction de déshydratation qui sont des matériaux homogènes ou multiphases solides insolubles dans le milieu réactionnel qui, bien qu'acides, pouvaient présenter aussi certains sites indésirables vraisemblablement à l'origine de la formation des sous-produits par des mécanismes réactionnels parfois peu prévisibles.

Le but de la présente invention est de pallier ces inconvénients en mettant en oeuvre le procédé en ajoutant dans le milieu gazeux réactionnel un composé susceptible de se fixer, au moins temporairement, sur ces sites et en les inhibant en cours de processus d'éviter la formation des sous-produits.

La présente invention a pour objet un procédé de synthèse de l'acroléine par déshydratation du glycérol en présence d'un catalyseur acide solide caractérisé en ce qu'il est mis en oeuvre dans un milieu réactionnel comportant une phase gazeuse contenant un composé acide.

On entend par composé acide, au sens de la présente invention, un composé qui, outre ce qui sera précisé ci-après, présentera en solution dans l'eau un pKa inférieur à 6,3. En particulier, le CO₂ n'est pas un acide au sens de la présente invention.

La réaction de déshydratation est effectuée par exemple sur des catalyseurs solides acides tels que ceux décrits dans le brevet français FR 2.882.052.

Les catalyseurs qui conviennent sont des matériaux homogènes ou multiphases, insolubles dans le milieu réactionnel qui ont une acidité de Hammett, notée H₀ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse. Les catalyseurs répondant au critère d'acidité H₀ inférieur à +2, peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes, des phosphates de fer ou encore des hétéropolyacides.

Avantageusement, les catalyseurs sont choisis parmi les zéolithes, les composites Nafion® (à base d'acide sulfonique de polymères fluorés), les alumines chlorées, les acides et sels d'acides phospho ou silico-tungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silicoaluminate SiO₂Al₂O₃, imprégnés de fonctions acides telles que borate BO₃, sulfate SO₄, tungstate WO₃, phosphate PO₄, silicate SiO₂, ou molybdate MoO₃. Selon les données de la littérature, ces catalyseurs ont tous une acidité de Hammett H₀ inférieure à +2.

Les catalyseurs préférés sont les zircones sulfatées, les zircones phosphatées, les zircones tungstées, les zircones silicées, les oxydes de titane ou d'étain sulfatés, les alumines ou silices phosphatées, les phosphates de fer dopés, les sels d'acide phospho ou silico-tungstiques.

Ces catalyseurs ont tous une acidité de Hammett H₀ inférieure à +2, l'acidité H₀ peut alors varier dans une large mesure, jusqu'à des valeurs pouvant atteindre -20 dans l'échelle de référence avec les indicateurs de Hammett. Le tableau donné à la page 71 de la publication sur la catalyse acido-basique (C. Marcilly) Vol 1 aux Editions Technip (n°ISBN 2-7108-0841-2) illustre des exemples de catalyseurs solides dans cette gamme d'acidité.

Les catalyseurs sélectionnés pour cette réaction sont des solides acides. L'acidité des solides peut être mesurée de nombreuses façons et la méthode de Hammett n'est que l'une d'entre elles.

L'ouvrage de C. Marcilly référencé ci-dessus liste par ailleurs différentes méthodes de mesure de l'acidité et de la basicité des solides.

On citera les publications de Aline Auroux où sont décrites différentes méthodes de mesure des échelles d'acidité des solides telles que : A. Auroux et A. Gervasini, « J. Microcalorimetric Study of the Acidity and Basicity of Metal Oxide Surfaces » Phys. Chem., (1990) 94, 6371-79 et L. Damjanovic et A. Auroux, dans « Handbook of Thermal Analysis and Calorimetry », Vol 5, Chapter 11 pages 387-485 : Recent Advances, Techniques and Applications, M.E. Brown et P.K. Gallager, editors (2008 Elsevier B.V.).

Des méthodes utilisées pour mesurer cette acidité sont décrites dans les brevets EP 1 714 696 [0038et 0039] et EP 1 714 955 [0045et 0046] où l'on distingue les cas où le solide est de couleur blanche ou non.

Ces travaux illustrent notamment qu'un solide est rarement constitué soit de sites uniquement acides, soit de sites uniquement basiques. Les solides acides ont la plupart du temps à la fois des sites acides, majoritaires, mais aussi certains sites basiques. Cette dichotomie est particulièrement illustrée dans l'article de A. Auroux A. Gervasini à la page 6377 où la figure 13 montre qu'un même oxyde peut à la fois adsorber un composé acide tel que CO₂ et un composé basique tel que NH₃. Sans vouloir se lier à une quelconque théorie, on pense que ces derniers contribuent à la formation des sous produits dans le procédé.

Le procédé est mis en oeuvre en présence d'un composé acide présent dans la phase gazeuse du milieu réactionnel et qui présente une affinité avec les sites indésirables basiques constituant le catalyseur. Ce composé sera choisi parmi les acides durs et mous tels qu'ils sont définis dans la classification dite de Pearson illustrée dans les articles suivants : R.G. Pearson, J. Am. Chem. Soc., 85, 3533 (1963) ; R.G. Pearson, Science, 151 (1966)172 ; R.G. Pearson, Chemistry in Britain, March 1967, 103 ; R.G. Pearson, J. Chemical Education, Vol45 N°9 (1968) 581 et Vol 45 N°10 (1968) 643; R.G. Parr and R.G. Pearson, J. Am. Chem. Soc, (1983) 105, 7512.

Il faut souligner que dans l'ouvrage de C Marcilly référencé plus haut aux pages 34 et suivantes est utilisée l'échelle fondée sur la théorie de Pearson.

Ces composés peuvent être gazeux aux conditions normales mais ils peuvent être soit liquides ou même solides s'ils sont susceptibles de passer, dans les conditions opératoires du procédé, dans la phase gazeuse du milieu réactionnel.

De préférence, la déshydratation est conduite en présence d'une phase gazeuse contenant une fraction mineure d'au moins un composé acide au sens de la classification de Pearson

Ce composé acide sera choisi notamment parmi SO₃, SO₂, NO₂, etc... On ne sortirait pas du cadre de l'invention, si l'on utilise un mélange de ces composés. Selon la théorie de Pearson les acides durs préfèrent s'associer aux bases dures et les acides mous aux bases moles. On peut utiliser un mélange de composés combinant différentes acidités afin d'inhiber les différents sites basiques présents sur le catalyseur.

La teneur en composés acides dépendra de la nature du catalyseur choisi pour la réaction de déshydratation. Elle sera généralement comprise entre 1 et 3000 ppm de la phase gazeuse ou exprimée en pourcentages volumiques de 0.0001 à 0.3 %.

Si la réaction est effectuée en phase liquide, le composé acide peut être sous forme liquide ou même solide dès lors qu'il est susceptible dans les conditions réactionnelles de passer en phase liquide pour atteindre les teneurs ci-dessus ou, en cas de composé solide, de se solubiliser puis de passer en phase liquide ainsi que cela a été précisé ci-dessus.

Il est à noter que le brevet EP 1 253 132 décrit l'utilisation dans un procédé de synthèse de l'acide acrylique par oxydation d'alcanes ou d'acroléine en présence d'un composé réducteur constitué par des acides organiques (acide formique ou oxalique) ou des composés contenant du soufre tels que SO₂ ou H₂S, SO₂ étant préféré. Cependant on peut souligner qu'il ne s'agit pas de la même réaction avec un catalyseur différent et que l'activité dudit composé est de stabiliser le catalyseur et non pas d'augmenter sa sélectivité. La réaction selon l'invention peut être mise en oeuvre en phase gaz ou en phase liquide, de préférence en phase gaz.

Lorsque la réaction est réalisée en phase gaz, différentes technologies de procédé peuvent être utilisées, à savoir procédé en lit fixe, procédé en lit fluidisé ou procédé en lit fluidisé circulant. Dans les 2 premiers procédés, en lit fixe ou en lit fluidisé, la régénération du catalyseur peut être séparée de la réaction.

Elle peut se faire ex-situ, par exemple par extraction du catalyseur et combustion sous air ou avec un mélange gazeux contenant de l'oxygène moléculaire. Dans ce cas, la température et la pression auxquelles est opérée la régénération n'ont pas besoin d'être les mêmes que celles auxquelles est effectuée la réaction. De préférence l'ajout des composés acides au sens de Pearson, est effectué au niveau du Réacteur et non au cours de la régénération.

Selon le procédé de l'invention, elle peut se faire en continu in-situ, en même temps que la réaction compte tenu de la présence d'une faible quantité d'oxygène moléculaire ou d'un gaz contenant de l'oxygène moléculaire dans le réacteur. Dans ce cas, la régénération s'apparente à une inhibition de la désactivation et se fait à la température et à la pression de la réaction. Du fait de ces conditions particulières où la régénération a lieu en continu, l'injection du composé acide gazeux se trouve être simultanée et de préférence en amont du lit catalytique de sorte que les composés acides soient parfaitement mélangés dans le mélange réactionnel.

Dans le procédé à lit fluidisé circulant, le catalyseur circule dans deux capacités, un réacteur et un régénérateur. On sait que la réaction de déshydratation est endothermique, il faut donc apporter de l'énergie à la première capacité, alors que la régénération consistant en la combustion du coke est exothermique, il faut donc extraire des calories de la deuxième capacité. Dans le cas du lit fluidisé circulant, les deux systèmes peuvent se compenser : selon le procédé de l'invention, la régénération du catalyseur sous flux d'oxygène par combustion conduit à un réchauffement du catalyseur et par conséquent fournit l'énergie nécessaire pour la réaction de déshydratation lorsque le catalyseur réchauffé retourne dans le réacteur. Le temps de séjour dans chaque capacité dépend de la vitesse de désactivation du catalyseur et du taux de coke formé sur le catalyseur. En effet, un taux de coke minimal est souhaitable pour pouvoir ramener le solide à la bonne température, et un taux de coke maximal est nécessaire pour éviter de dégrader le solide par frittage lors de la combustion. L'injection du composé acide gazeux est de préférence effectuée au Réacteur.

La réaction de déshydratation est effectuée en phase gaz en présence d'un catalyseur à une température allant de 150°C à 500°C, de préférence comprise entre 250°C et 350°C, et une pression comprise entre 1 et 5 bars. Lorsque la réaction est effectuée en phase liquide en présence d'un catalyseur à une température allant de 150°C à 500°C, de préférence comprise entre 250°C et 350°C, et une pression supérieure à 5 bars et de préférence comprise entre 20 et 80 bars.

Les exemples suivants illustrent le procédé de la présente invention

Lors de la déshydratation du glycérol en présence d'un catalyseur acide classique on obtient de l'acroléine mais aussi des sous-produits, tels que l'hydroxypropanone, le propanaldéhyde, l'acétaldéhyde, l'acétone, le phénol, les produits d'addition de l'acroléine sur le glycérol, les produits de polycondensation du glycérol, des éthers de glycérol cycliques ou non.

Ces exemples vont illustrer l'incidence de la présence du composé acide sur la sélectivité de la réaction au regard des divers sous-produits connus et notamment de l'hydroxypropanone qui est le composé le plus présent et est donc représentatif de l'efficacité du procédé. Ils illustreront également l'incidence de la présence de composés acide sur la désactivation du catalyseur.

### Exemple 1 :

La réaction peut être conduite dans les conditions suivantes. On utilise un réacteur en pyrex contenant un lit de catalyseur retenu par un fritté. On charge tout d'abord un catalyseur tel que le catalyseur de déshydratation Zircone Tungstée de DaiIchi Kigenso KK, de référence Z1044 ayant une masse de 6,6 g environ et réduit à une granulométrie de 0,1-0,15 mm dilué avec 7 ml de carbure de silicium de fine granulométrie (0,125 mm). Ensuite on charge, une série de lits de carbure de silicium de granulométries différentes : 2ml en 0,125 mm , 7 ml en 0,5 mm et enfin 1,19 mm jusqu'en haut du réacteur.

Le réacteur est ensuite placé dans un four connecté à l'installation de test. La température du catalyseur est régulée en température à 305 °C mesurée au niveau de la « couche de déshydratation ».

Le réacteur est alimenté par le haut par un mélange gazeux de Hélium-Krypton / SO₂ / Eau-Glycérol à une pression de 1,3 bar absolus. Le mélange gazeux Hélium-Krypton contient 4,92 % de Krypton qui sert d'étalon interne. Le mélange eau-glycérol contient 30 % en poids de glycérol.

La constitution du mélange injecté est la suivante exprimée en pourcentage molaire :
Hélium/Krypton/O₂/SO₂/ Eau/Glycérol : *50* / *2,6* / *3,4* / *0,02* / *40,6* / *3,4.*

Le débit d'introduction du mélange de charge est tel que la vitesse volumique horaire (VVH) sera de 2 000 h⁻¹. . La vitesse volumique horaire est égale au ratio du débit gazeux total du mélange gazeux exprimé en normaux litres par heure par le volume apparent de catalyseur exprimé en litres.

Les effluents sont piégés dans l'eau en sortie du réacteur par un piège refroidi à 0°C permettant de séparer les effluents liquides des incondensables. L'acroléine et l'hydroxypropanone, comme composé modèle des sous produits autre que l'acide acrylique, sont dosés par analyse chromatographique.

Les effluents sont cumulés dans le piège pendant une durée de 60 minutes. Les gaz non condensables sont analysés pendant la durée du bilan. Le rendement en acroléine produite est de 70 mole %, et en acide acrylique de 2 mole % et en hydroxyacétone de 0,5 mole %.

### Exemple 2 (Comparatif)

On reproduira l'exemple 1, mais en absence de SO₂.

Les effluents sont cumulés dans le piège pendant une durée de 60 minutes . Les gaz non condensables sont analysés pendant la durée du bilan. Le rendement en acroléine produite est de 68 mole %, et en acide acrylique de 2 mole % et en hydroxyacétone de 2 mole %.

### Exemple 3 :

On utilise le même réacteur en pyrex qu'à l'exemple 1. Il est chargé avec un catalyseur de déshydratation zircone tungstée de Daiichi Kigenso Kagaku Kogyo de référence Z1044 ring, broyé et tamisé à une granulométrie de 0,32-0,50 mm, de volume 7 ml et masse 9,18 g. Le catalyseur non dilué est placé entre 2 couches de carbure de silicium.

Le réacteur est placé dans un four qui est régulé à une température de 275°C. Le réacteur est alimenté par un mélange gazeux à 275°C de N₂/O₂/SO₂/eau/glycérol à une pression de 1,3 bar absolus. Ce mélange gazeux est obtenu en injectant dans un vaporiseur électrique d'une part un courant d'azote et un courant d'oxygène contrôlés en débit par des régulateurs de débit massique et d'autre part un flux liquide d'un mélange glycérol (Prolabo), eau déminéralisée et acide sulfureux à 7,4% SO₂ (Sigma-Aldrich) par une pompe volumétrique de type HPLC et dont le débit est contrôlé par une balance.

La constitution du mélange injecté est la suivante exprimée en pourcentages molaires :
N₂/O₂/SO₂/eau/glycérol : *15,4*/*3,9*/*0,005*/*74,5*/*6,2.*

Le débit d'introduction du mélange de charge est tel que la vitesse volumique horaire (VVH) est de 4 200 h⁻¹

Après 3 heures d'injection du mélange gazeux sur le catalyseur, on réalise un bilan matière pendant 90 minutes de la même façon qu'à l'exemple 1. Les résultats sont reportés au tableau 1.

### Exemple 4 :

Les conditions de l'exemple 3 sont reproduites avec un mélange gazeux de composition molaire :
N₂/O₂/SO₂/eau/glycérol : *15,4*/*3,9*/*0,025*/*74,5*/*6,2.*

On réalise un bilan après 3 heures et après 24 heures d'injection du mélange.

Les résultats sont reportés au tableau 1.

### Exemple 5 (Comparatif)

Les conditions de l'exemple 3 sont reproduites avec un mélange gazeux de composition molaire :
N₂/O₂/SO₂/eau/glycérol : *15,4*/*3,9*/ *0* /*74,5*/*6,2.*

On réalise un bilan après 3 heures et 24 heures.

Les résultats sont reportés au tableau 1.

**Tableau 1**

| Exemple | 3 | 4 | | 5 (comparatif) | |
|---|---|---|---|---|---|
| Durée d'injection (h) | 3 | 3 | 24 | 3 | 24 |
| SO₂ (mol %) | 0,005 | 0,025 | 0,025 | 0 | 0 |
| Conversion glycérol (%) | 100 | 100 | 87 | 100 | 69 |
| Rendement acroléine (%) | 73 | 73 | 60 | 72 | 49 |
| Rendement hydroxypropanone (%) | 0, 4 | 0,2 | 5, 9 | 2,4 | 5, 9 |

On constate que l'ajout de SO₂ induit non seulement une amélioration du rendement, mais également limite la désactivation du catalyseur.

## Revendications

1. Procédé de synthèse de l'acroléine par déshydratation du glycérol en présence d'un catalyseur acide solide ayant une acidité de Hammett inférieure à + 2, **caractérisé en ce qu'**il est mis en oeuvre dans un milieu réactionnel comportant une phase gazeuse contenant une teneur comprise ente 1 et 3000 ppm d'au moins un composé acide au sens de la classification de Pearson choisi parmi SO₃, SO₂, NO₂.

2. Procédé selon la revendication 1 **caractérisé en ce que** le catalyseur est choisi parmi les zéolithes, les composites Nafion®, les alumines chlorées, les acides et sels d'acides phospho ou silico-tungstiques et/ou silicotungstiques, et les solides de type oxydes métalliques tels que oxyde de tantale Ta₂O₅, oxyde de niobium Nb₂O₅, alumine AlₛO₃, oxyde de titane TiO₂, zircone ZrO₂, oxyde d'étain SnO₂, silice SiO₂ ou silicoaluminate SiO₂-Al₂O₃, imprégnés de fonctions acides telles que borate BO₃, sulfate SO₄, tungstate WO₃, phosphate PO₄, silicate SiO₂, ou molybdate MoO₃.

3. Procédé selon la revendication 1 ou 2 **caractérisé en ce que** le catalyseur est choisi parmi les zircones sulfatées, les zircones phosphatées, les zircones tungstées, les zircones silicées, les oxydes de titane ou d'étain sulfatés, les alumines ou silices phosphatées, les phosphates de fer dopés, les sels d'acide phospho ou silico-tungstiques.

4. Procédé selon l'une des revendications 1 à 3 **caractérisé en ce que** la réaction de déshydratation est effectuée en phase gaz à une température comprise entre 150°C et 500°C, de préférence comprise entre 250°C et 350°C, et sous une pression comprise entre 1 et 5 bars.

5. Procédé selon l'une des revendications 1 à 3 **caractérisé en ce que** la réaction est effectuée en phase liquide à une température comprise entre 150°C et 500°C, de préférence comprise entre 250°C et 350°C, et sous une pression supérieure à 5 bars et de préférence comprise entre 20 et 80 bars.

## Patentansprüche

1. Verfahren zur Synthese von Acrolein durch Dehydratisierung von Glycerin in Gegenwart eines festen Säurekatalysators mit einer Hammett-Azidität von unter + 2, **dadurch gekennzeichnet, dass** es in einem Reaktionsmedium durchgeführt wird, das eine Gasphase mit einem Gehalt von zwischen 1 und 3000 ppm mindestens einer sauren Verbindung im Sinne der Klassifizierung von Pearson, ausgewählt aus SO₃, SO₂, NO₂, enthält.

2. Verfahren nach Anspruch 1, dadurch gekenntzeichnet, dass der Katalysator ausgewählt ist aus Zeolithen, Nafion®-Kompositen, chlorierten Aluminiumoxiden, den Säuren und Säuresalzen von Phosphorwolfram- und/oder Kieselwolframsäuren und Feststoffen des Metalloxid-Typs, wie Tantaloxid Ta₂O₅, Nioboxid Nb₂O₅, Aluminiumoxid Al₂O₃, Titanoxid TiO₂, Zirkoniumdioxid ZrO₂, Zinnoxid SnO₂, Siliziumdioxid SiO₂ oder Alumosilikate SiO₂-Al₂O₃, die mit Säurefunktionen, wie Borat BO₃, Sulfat SO₄, Wolframat WO₃, Phosphat PO₄, Silikat SiO₂ oder Molybdat MoO₃, imprägniert sind.

3. Verfahren nach Anspruch 1 oder 2, **dadurch gekennzeichnet, dass** der Katalysator ausgewählt wird aus Sulfat-Zirkoniumdioxiden, Phosphatzirkoniumdioxiden, Wolfram-Zirkoniumdioxiden, Kieselsäure-Zirkoniumdioxiden, Sulfat-Titanoxiden oder Sulfat-Zinnoxiden, Phosphat-Aluminiumoxiden oder Phosphat-Siliziumdioxiden, dotierten Eisenphosphaten, Salzen von Phosphorwolfram- und/oder Kieselwolframsäure.

4. Verfahren nach einem der Ansprüche 1 bis 3, **dadurch gekennzeichnet, dass** die Dehydratisierungsreaktion in der Gasphase bei einer Temperatur zwischen 150°C und 500°C, vorzugsweise zwischen 250°C und 350°C, und unter einem Druck zwischen 1 und 5 bar durchgeführt wird.

5. Verfahren nach einem der Ansprüche 1 bis 3, **dadurch gekennzeichnet, dass** die Reaktion in der Flüssigphase bei einer Temperatur zwischen 150°C und 500°C, vorzugsweise zwischen 250°C und 350°C, und unter einem Druck von mehr als 5 bar und vorzugsweise zwischen 20 und 80 bar durchgeführt wird.

## Claims

1. A process for the synthesis of acrolein by dehydration of glycerol in the presence of a solid acid catalyst having a Hammett acidity of less than +2, **characterized in that** it is implemented in a reaction medium comprising a gas phase comprising a content between 1 and 3000 ppm of at least one acid compound within the meaning of the Person classification chosen from SO₃, SO₂ or NO₂.

2. The process as claimed in claim 1, **characterized in that** the catalyst is chosen from zeolites, Nafion® composites, chlorinated aluminas, phosphotungstic and/or silicotungstic acids and acid salts, and solids of the type comprising metal oxides, such as tantalum oxide Ta₂O₅, niobium oxide Nb₂O₅, alumina Al₂O₃, titanium oxide TiO₂, zirconia ZrO₂, tin oxide SnO₂, silica SiO₂ or silicoaluminate SiO₂/Al₂O₃, impregnated with acid functional groups, such as borate BO₃, sulfate SO₄, tungstate WO₃, phosphate PO₄, silicate SiO₂ or molybdate MoO₃.

3. The process as claimed in claim 1 or claim 2, **characterized in that** the catalyst is chosen from sulfated zirconias, phosphated zirconias, tungstated zirconias, silica zirconias, sulfated titanium or tin oxides, phosphated aluminas or silica, doped iron phosphates, or phosphor- or silicotungstic acid salts.

4. The process as claimed in one of claims 1 to 3, **characterized in that** the dehydration reaction is carried out in the gas phase at a temperature of between 150°C and 500°C, preferably of between 250°C and 350°C, and under a pressure of between 1 and 5 bar.

5. The process as claimed in one of claims 1 to 3, **characterized in that** the reaction is carried out in the liquid phase at a temperature of between 150°C and 500°C, preferably of between 250°C and 350°C, and under a pressure of greater than 5 bar and preferably of between 20 and 80 bar.
